# EUROPEAN PATENT SPECIFICATION

(11) **EP 1 174 084 B1**
(45) Date of publication and mention of the grant of the patent: **18.06.2003**
(21) Application number: 01126654.1
(22) Date of filing: 03.03.1998
(51) Int. Cl.: A61B 5/15, B65D 41/04

(54) **Assembly for collecting blood or other body fluids**
Vorrichtung zum Sammeln von Blut oder anderen Körperflüssigkeiten
Dispositif pour collecter du sang ou d'autres fluides corporels

(30) Priority: 26.03.1997 US 42062 P
(43) Date of publication of application: 23.01.2002
(62) Divisional of application: 98103704.7
(73) Proprietor: Becton Dickinson and Company, Franklin Lakes, New Jersey 07417-1880 (US)
(72) Inventor: Newby, Mark C., Tuxedo, New-York 10987 (US); Miller, Henry F., Clifton, New Jersey (US); Schleiffer, Keith E., Gahanna, Ohio 43230 (US)
(74) Representative: Selting, Günther, Dipl.-Ing.

(56) References cited:
- WO-A-81/01238
- US-A- 5 061 263

## Description

The field of the invention relates to assemblies for collecting blood or other body fluids, the assemblies being of the type including a container having a closure assembly pierceable by a needle or cannula.

Blood collection tubes are commonly used by doctors, nurses and other persons to draw a sample of a body fluid from a patient or to receive a fluid sample from another vessel. Such tubes are ordinarily evacuated, and include a pierceable closure. During one typical use of a blood collection tube, one end of a double-ended needle assembly is used to pierce a vein. The evacuated blood collection tube is then urged towards the second end of the double-ended needle assembly until its closure is pierced. Blood is thereby drawn into the tube.

The double-ended needle assembly is ordinarily mounted to a holder having a tubular body. The blood collection tube is inserted within the tubular body in order to engage the second end of the double ended needle assembly.

The maintenance of a vacuum within the blood collection tube is important. Blood collection tubes may be stored for considerable lengths of time before they are used. Loss of vacuum during storage or at any time can render the collection tube less effective or useless.

A number of designs of blood collection tubes and their closures have been the subjects of patents and other disclosures. British Patent 1 388 494 and U.S. Patent No. 4,327,746 both disclose closures for such tubes having sealing membranes positioned between a cap and the open end of the collection tube. U.S. Patent No. 5,061,263 discloses several closure designs, each including a cap, a gas barrier member and a re-sealable member.

An assembly for collecting blood or other body liquids, according to the precharacterizing part of claim 1, is disclosed in WO 81 01238 A.

U.S. Patent Nos. 4,150,666, 4,967,919, 4,991,601, 5,326,534, 5,494,170 and EP 0 513 901 A disclose various blood collection tubes and/or holders for such tubes. The '170 patent discloses a cap having flexible tabs which engage the wall of the holder, thereby maintaining the blood collection tube within the holder.

It is an object of the present invention to provide a body fluid collection assembly which includes a closure assembly that is both easily removed and easily reinstalled.

The present invention is defined by Claim 1.

Further, preferred embodiments are disclosed in dependent claims 2 and 3.

The open end of the container includes external interrupted threads. The cap includes a plurality of internal threads, each of which includes a lead portion. Internal tabs are provided adjacent each of the lead portions for allowing the user to easily locate the cap for reapplication to the open end of the container. The threads preferably allows the cap to be removed upon less than 180° rotation so that such removal can be accomplished using only one hand.
Fig. 1 is a perspective view of an assembly for collecting body fluids and a tube holder capable of receiving a portion of the assembly;
Fig. 2 is a top perspective view showing the assembly having an end portion positioned within the tube holder;
Fig. 3 is a sectional elevation view showing the assembly for collecting body fluids and the tube holder;
Fig. 4 is a sectional elevation view showing the assembly positioned within the tube holder;
Fig. 5 is an enlarged, sectional view showing the assembly fully inserted within the tube holder;
Fig. 6 is an exploded, perspective view of the assembly for collecting body fluids;

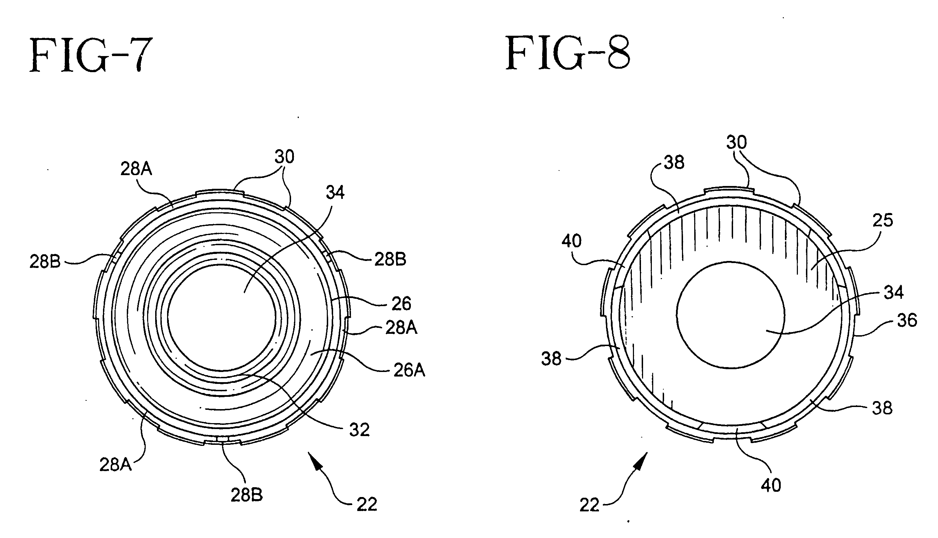

Fig. 7 is a top plan view of a cap of the assembly for collecting body fluids;
Fig. 8 is a bottom plan view thereof, and
Fig. 9 is a bottom perspective view thereof.

### DETAILED DESCRIPTION OF THE INVENTION

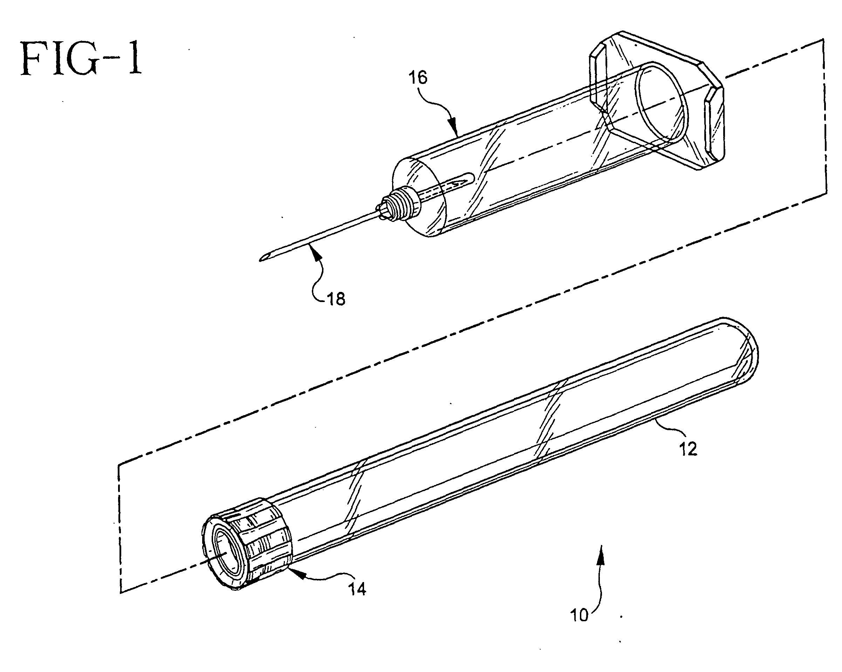

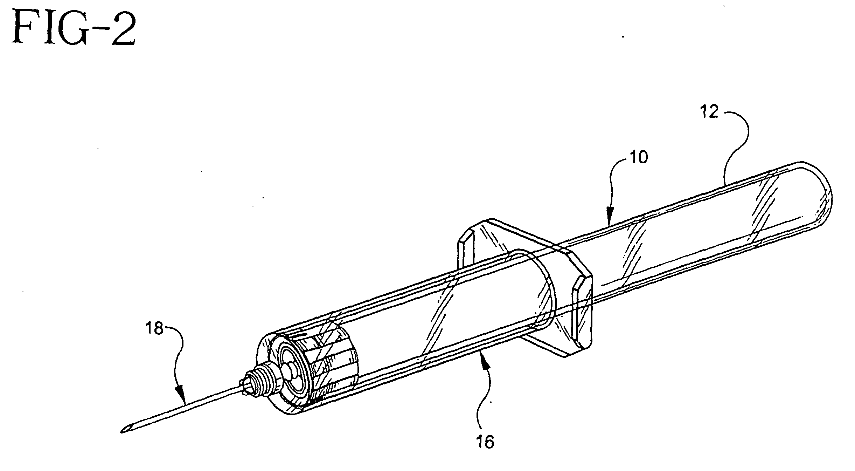

An assembly 10 for collecting blood or other body fluids is provided by the invention. Referring to Figs. 1-6, the assembly includes a generally cylindrical container 12 having a closed end and an open end, and a closure assembly 14 which can be removably mounted to the open end of the container. As shown in Figs. 1-5, the container may be employed in conjunction with a holder 16 to which a double-ended needle assembly 18 is mounted.

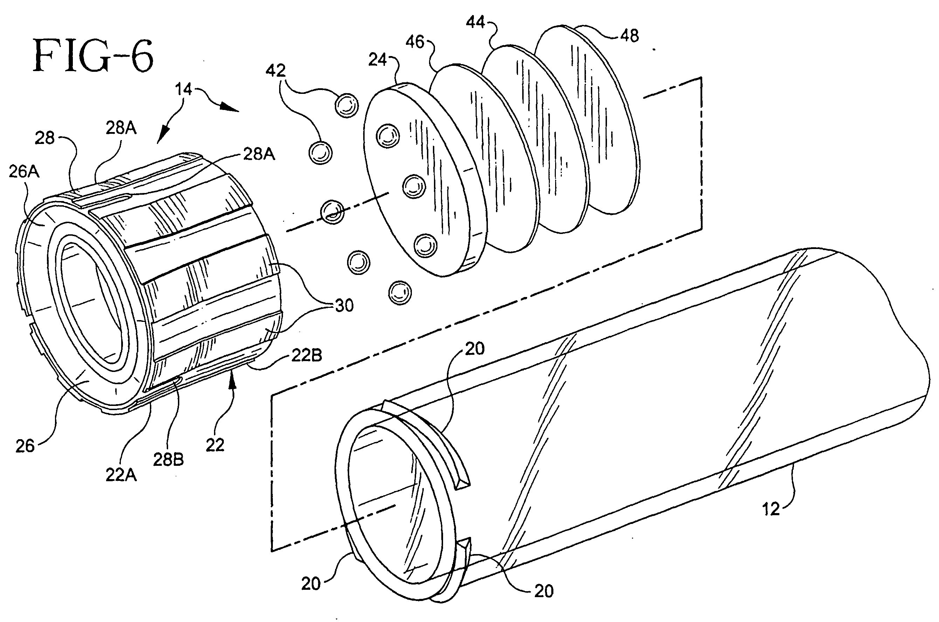

The container 12 is preferably transparent, and is preferably made from a material that is substantially gas-impermeable, such as glass or polyethylene terephthalate (PET). As shown in Fig. 6, the container includes an interrupted thread including three thread segments 20. Each thread segment has a first end portion adjoining the rim and a second end portion displaced spirally downwardly with respect to the first end portion. A gap separates each thread segment. The depth of the interrupted thread shown in the drawings is less than about 1,8 mm (0.070 inches)

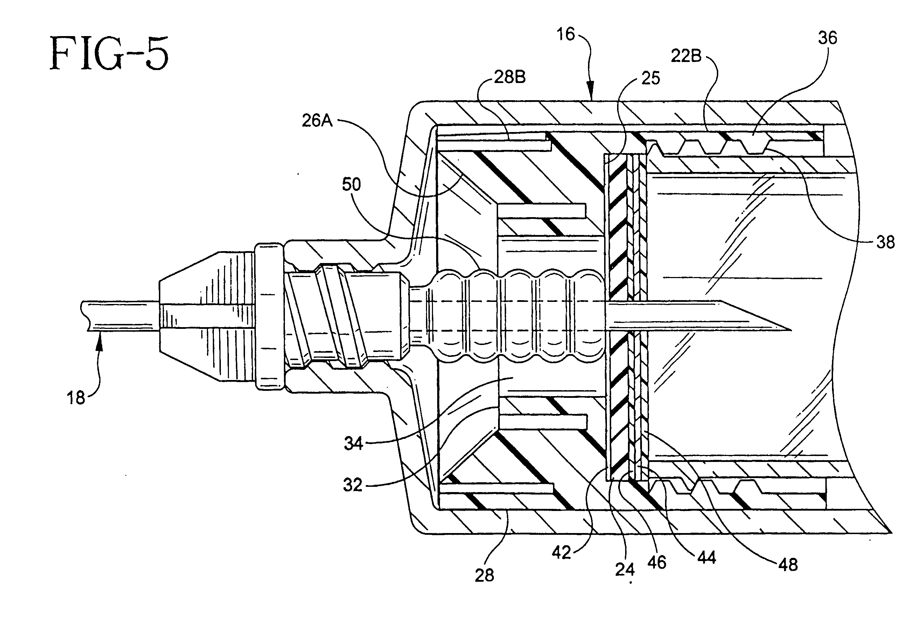

The closure assembly 14 is most clearly illustrated in Figs. 5 and 6. It includes a generally cylindrical cap 22 made of polypropylene or other semi-rigid polyolefin material and a resealable member 24. The resealable member is made from an elastomeric material such as polyurethane or, alternatively, butyl rubber, which is capable of resealing itself following piercing of the same by a needle and subsequent needle withdrawal.

The cap 22 includes a top portion 22A and a bottom portion 22B. An annular shoulder 25 is defined by the bottom surface of the top portion of the cap. The top portion of the cap further includes an annular, substantially rigid body portion 26 surrounded by an annular deflectable portion 28. The deflectable portion 28 is comprised of a plurality of arcuate walls 28A separated by slots 28B extending downwardly from the upper rim of the deflectable portion. The outer surface of the cap includes a plurality of elongate, radially extending protrusions 30. These protrusions facilitate the handling of the cap and assembly 10. They also provide contact surfaces in the event the assembly is dropped. Force is thereby imparted to the deflectable portion 28 of the cap.

The body portion 26 of the upper portion of the cap includes a chamfered upper surface 26A, which may include indicia such as opening and closing instructions. An annular wall 32 is positioned inwardly of the body portion 26. The inner surface of this annular wall 32 defines an axial opening 34 extending through the upper portion of the cap.

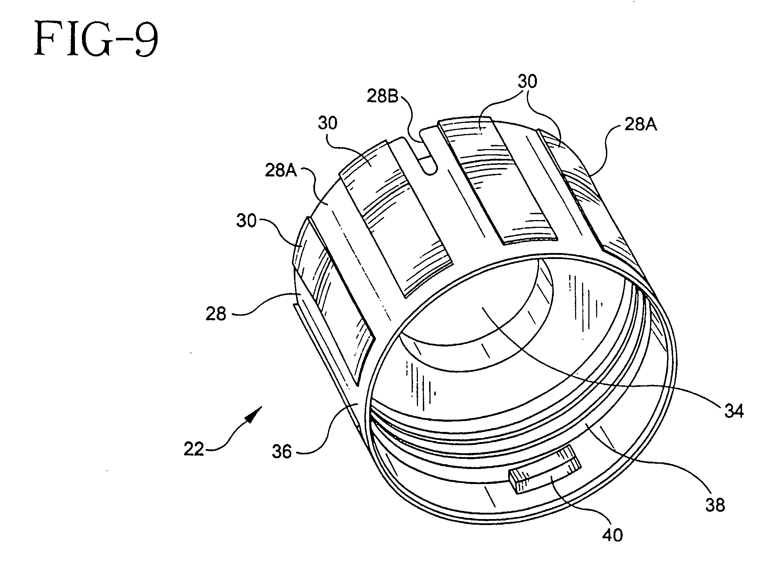

The bottom portion 22B of the cap 22 is defined by a substantially cylindrical wall 36 including a plurality of internal threads 38. In the embodiment shown herein, three spiral threads are provided, each having a thread lead adjoining a locating tab 40. (See Fig. 9). The locating tabs extend axially towards the bottom of the cap. The width of each tab is smaller than the width of each gap which separates the external thread segments 20 of the container 12. Each thread 38 extends spirally for 180° or less, preferably about 120-140°. This allows the cap 22 to be mounted to or removed from the container by turning it 180° or less. Such a task does not ordinarily require a two-handed operation. The cap can alternatively be snapped on the container. This feature facilitates the manufacturing process. The locating tabs facilitate rethreading the cap onto the container subsequent to its initial removal.

The resealable member 24 is shown in the drawings as a flat disc. This particular configuration is not critical, though a relatively thin center portion does facilitate needle penetration. The member 24 is secured to the shoulder 25, thereby closing the opening 34 through the upper portion of the cap 22. Hot melt adhesive 42 or other suitable adhesive may be used to permanently secure the resealable member to the cap.

A gas barrier member 44 is secured to the resealable member 24 by a tie layer 46 of adhesive material or a material such as PET. The gas barrier member is also bonded to the top portion of the container, and preferably the rim, by a seal layer 48 which may also be PET. (See Figs. 5 and 6). A seal layer which allows induction sealing is preferred. It should be understood that the drawings of the resealable member, gas barrier member and other sealing members are not to scale. The gas barrier member is preferably a metal foil, such as aluminum foil, having a thickness of about 0.001 inch. The tie and seal layers 46, 48 may be coatings on the gas barrier member.

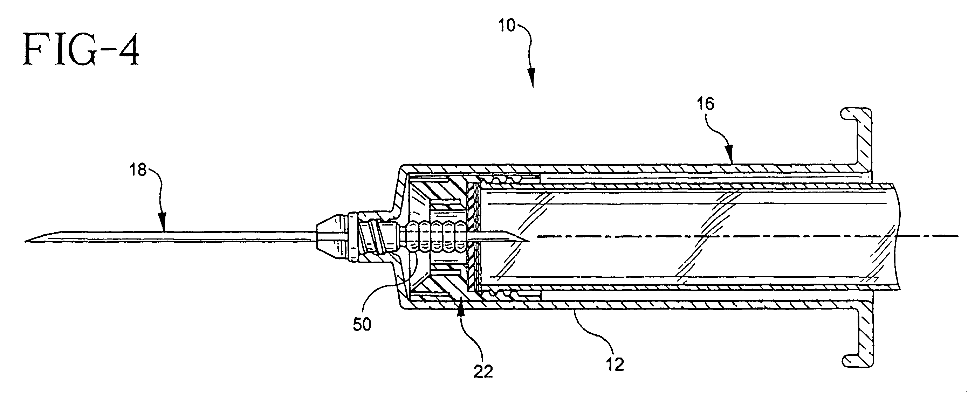

The assembly 10 as described above may be used with a holder 16 as shown in the drawings or other holders known to the art. As shown in Fig. 4, the assembly 10 is used to collect blood or other body fluid when inserted within the holder such that the rear end of the double-ended needle assembly 18 extends through the resealable member 24 and gas barrier member 44. The figures show a sheath 50 which is displaced upon insenion of the assembly 10 into the holder. The use of such a sheath is not required.

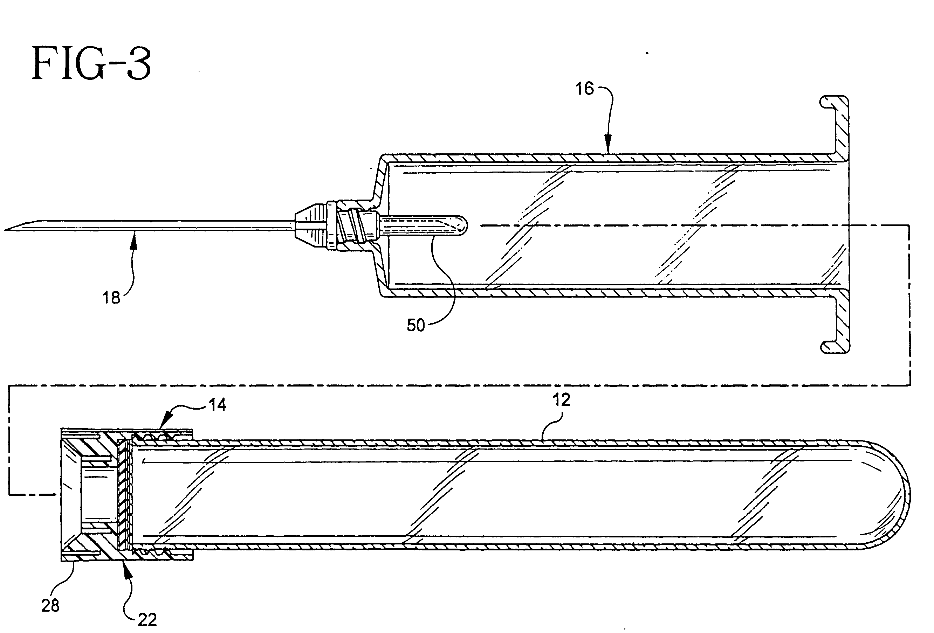

The assembly 10 is provided to users in the form shown in Figs. 1 and 3. The container 12 is ordinarily at least partially evacuated. As discussed above, the assembly 10 is designed to maintain a desired vacuum for a considerable length of time. The product should accordingly have a satisfactory shelf life.

Once the fluid collection procedure is completed, the assembly 10 is withdrawn from the holder 16. Though the closure assembly 14 is no longer gas-impermeable following such withdrawal, liquid impermeability is maintained by the resealable member. The assembly 10 can then be transported to an area where the fluid content of the container can be analyzed.

Removal of the closure assembly 14 is a relatively simple procedure which can be accomplished with one hand. The cap 22 is rotated about 120-140° in accordance with the preferred embodiment of the invention, and is then moved away from the open end of the container. The contents of the container can then be accessed. The closure assembly can be secured again to the open end of the container by first placing the cap over this end and then rotating it. The locating tabs 40 greatly facilitate the engagement of the container threads 20 and the cap threads 38.

Because the seal between the gas barrier member 44 and the container is relatively weak, this element preferably suffers no damage as the cap is turned and ultimately removed. The cap, resealable member and gas barrier member are accordingly removed as a unit and reapplied as a unit. The gas barrier member, being intact, forms a liquid-tight seal with the top rim of the container when the closure assembly is reapplied.

While the invention has been described with respect to the preferred embodiment thereof, it will be appreciated that alternative approaches may be taken with respect to the structures of the cap, sealing elements, and container. The threads employed for engaging the cap and container could, for example, be replaced by other engagement structures such as a bayonet locking arrangement (not shown).

## Claims

1. An assembly for collecting blood or other body liquids, comprising:
a container (12) having an open end and a closed end,
a closure assembly (14) for said container (12), said closure assembly including a cap (22) having an opening (34) therein and a resealable member (24) aligned with said opening; wherein said open end of said container (12) includes external, threads (20), and
said cap (22) includes a plurality of internal threads (38), each of said internal threads including a lead portion,
**characterised in that**
said cap further includes locating tabs (40) positioned, respectively, adjacent said lead portions of said internal threads, and said external threads (20) are interrupted.

2. An assembly as described in Claim 1, wherein said cap includes a top portion (22A), said opening (34) extending through said top portion, said resealable member (24) being bonded to said top portion, said closure assembly further including a gas barrier member (44) bonded to said resealable member (24) and to said open end of said container (12).

3. An assembly as described in Claims 1 or 2, wherein said threads (38,20) of said closure assembly (14) and container (12) are formed such that rotation of said closure assembly of less than 180° is sufficient to disengage said closure assembly (14) from said container (12) even when said closure assembly is fully threadably engaged to said open end of said container (12).

## Patentansprüche

1. Vorrichtung zum Sammeln von Blut oder anderen Körperflüssigkeiten, mit:
einem Behälter (12) mit einem offenen Ende und einem geschlossenen Ende,
einer Verschlussvorrichtung (14) für den Behälter (12), die eine Kappe (22) mit einer darin ausgebildeten Öffnung (34) und ein mit der Öffnung ausgerichtetes wiederabdichtendes Element (24) aufweist, wobei das offene Ende des Behälters (12) mit Außengewindegängen (20) versehen ist, und
die Kappe (22) mehrere Innengewindegänge (38) jeweils mit einem Führungsteil aufweist,
**dadurch gekennzeichnet, dass**
die Kappe ferner Positioniervorsprünge (40) aufweist, die jeweils benachbart zu den vorderen Teilen der Innengewindegänge angeordnet sind, und
die Außengewindegänge (20) eine unterbrochene Konfiguration aufweisen.

2. Vorrichtung nach Anspruch 1, bei der die Kappe einen oberen Teil (22A) aufweist, durch den die Öffnung (34) verläuft, wobei das wiederabdichtende Element (24) mit dem oberen Teil verbondet ist und die Verschlussvorrichtung ferner ein mit dem wiederabdichtenden Element und dem offenen Ende des Behälters (12) verbondetes Gassperrelement (44) aufweist.

3. Vorrichtung nach Anspruch 1 oder 2, bei der die Gewindegänge (38,20) der Verschlussvorrichtung (14) und des Behälters (12) derart ausgebildet sind, dass eine Drehung der Verschlussvorrichtung um weniger als 180° ausreicht, um die Verschlussvorrichtung (14) von dem Behälter (12) abzunehmen, selbst wenn die Verschlussvorrichtung in vollständigem Gewindeeingriff mit dem offenen Ende des Behälters (12) steht.

## Revendications

1. Ensemble destiné à recueillir du sang ou d'autres liquides corporels comprenant :
un récipient (12) présentant une extrémité ouverte et une extrémité fermée ;
un ensemble de fermeture (14) pour ledit récipient (12), ledit ensemble de fermeture comprenant un capuchon (22) ayant dedans une ouverture (34) et un organe apte à être réétanchéifié (24) étant aligné avec ladite ouverture ;
**caractérisé en ce que**
ladite extrémité ouverte dudit récipient (12) comprend des filetages interrompus externes (20), et
ledit capuchon (22) comprend une pluralité de filetages internes (38), chacun desdits filetages internes comprenant une partie de guidage, ledit capuchon comprenant, en outre, des pattes de positionnement (40) positionnées respectivement adjacentes aux parties de guidage desdits filetages internes.

2. Ensemble selon la revendication 1, dans lequel ledit capuchon comprend une partie supérieure (22A), ladite ouverture (34) s'étendant à travers ladite partie supérieure, ledit organe apte à être réétanchifié (24) étant relié à ladite partie supérieure, ledit ensemble de fermeture comprenant, en outre, un élément formant barrière de gaz (44) relié audit organe apte à être réétanchifié (24) et à ladite extrémité ouverte dudit récipient (12).

3. Ensemble selon la revendication 1 ou 2, dans lequel lesdits filetages (38, 20) dudit ensemble de fermeture (14) et récipient (12) sont formés de manière que la rotation dudit ensemble de fermeture est inférieure à 180° est suffisante pour désengager ledit ensemble de fermeture (14) depuis ledit récipient (12) même quand ledit ensemble de fermeture est vissé pleinement dans ladite extrémité ouverte dudit récipient (12).
